# EUROPEAN PATENT APPLICATION

(11) **EP 4 691 375 A1**
(43) Date of publication of application: **11.02.2026**
(21) Application number: 24784095.2
(22) Date of filing: 25.03.2024
(51) Int. Cl.: A61B 17/00, A61B 17/12

(54) **OCCLUDING DEVICE AND OCCLUDING SYSTEM**

(30) Priority: 04.04.2023 CN 202310351611
(71) Applicant: Hangzhou Endonom Medtech Co., Ltd., Hangzhou, Zhejiang 311217 (CN)
(72) Inventor: GUO, Wei, Hangzhou, Zhejiang 311217 (CN); LIU, Xingzhi, Hangzhou, Zhejiang 311217 (CN); CHEN, Jie, Hangzhou, Zhejiang 311217 (CN); WANG, Yongsheng, Hangzhou, Zhejiang 311217 (CN)
(74) Representative: Mathys & Squire
(86) International application number: PCT/CN2024/083561
(87) International publication number: WO 2024/208010

(57) **Abstract**

An occlusion device and an occlusion system are provided in the disclosure. The occlusion system includes an occlusion device (100). The occlusion device (100) includes an occlusion assembly (110) and a locking assembly (120). The occlusion assembly (110) includes a distal occlusion portion (111), a waist portion (113), and a proximal occlusion portion (112). The waist portion (113) is connected between the distal occlusion portion (111) and the proximal occlusion portion (112). The locking assembly (120) includes a distal locking portion (121) and a proximal locking portion (122). The distal locking portion (121) is connected to the distal occlusion portion (111), and the proximal locking portion (122) is connected to the proximal occlusion portion (112). Any one of the distal locking portion (121) and the proximal locking portion (122) includes an elastically deformable member (130), at least a portion of the elastically deformable member (130) is elastically deformable, and locking and unlocking between the distal locking portion (121) and the proximal locking portion (122) are achieved through elastic deformation of the elastically deformable member (130). The elastically deformable member (130) includes a mesh structure.

## Description

The application claims priority to Chinese Patent Application No. CN202310351611.X, filed April 4, 2023, and entitled "OCCLUSION DEVICE AND OCCLUSION SYSTEM", the entire disclosure of which is incorporated herein by reference.

### TECHNICAL FIELD

This disclosure relates to the field of medical equipment technology, and in particular, to an occlusion device and an occlusion system.

### BACKGROUND

With the development of interventional therapy, lesions within human body can be treated using various implantable devices. For example, expandable stents can be used to treat vascular stenosis, and occluders can be used to occlude tears in internal organs. Exemplarily, when a lesion in an organ is a tear in a blood vessel, an occluder can be used to occlude the tear in the blood vessel. For example, the occluder can be used to treat aortic dissection (AD). AD refers to a pathological condition where blood in the aortic lumen enters the aortic media through a tear in the aortic intima, causing separation of the media and expansion of the media in a direction of a longitudinal axis of the aorta, thereby creating true and false lumens within the aorta.

Due to factors such as vascular pulsation or muscle contraction in human bodies, the fixation effect of the occluder on the blood vessel may be affected, leading to detachment. As a result, postoperative risks are increased and therapeutic effect is reduced. Therefore, how to enhance the firmness of the occluder positioning at the blood vessel during interventional therapy is key to the safe use of the occluder and a major research focus in this field.

### SUMMARY

Based on this, an occlusion device and an occlusion system are provided according to embodiments of the disclosure.

The occlusion device includes an occlusion assembly and a locking assembly. The occlusion assembly includes a distal occlusion portion, a waist portion, and a proximal occlusion portion. The waist portion is connected between the distal occlusion portion and the proximal occlusion portion. The locking assembly includes a distal locking portion and a proximal locking portion. The distal locking portion is connected to the distal occlusion portion, and the proximal locking portion is connected to the proximal occlusion portion. Any one of the distal locking portion and the proximal locking portion includes an elastically deformable member, at least a portion of the elastically deformable member is elastically deformable, and locking and unlocking between the distal locking portion and the proximal locking portion are achieved through elastic deformation of the elastically deformable member. The elastically deformable member includes a mesh structure.

The occlusion system includes the occlusion device, a first operating member connected to the distal locking portion, and a second operating member connected to the proximal locking portion. The second operating member is axially movable relative to the first operating member, to adjust a relative distance between the distal occlusion portion and the proximal occlusion portion, adjust a relative distance between the distal locking portion and the proximal locking portion, and adjust a degree of elastic deformation of the elastically deformable member.

Details of one or more embodiments of the disclosure will be provided in the following accompanying drawings and description. Other features, objectives and advantages of the present application will become obvious from the description, accompanying drawings and claims.

### BRIEF DESCRIPTION OF THE DRAWINGS

FIG. 1a is a schematic structural view of an occlusion device according to an embodiment of the disclosure.
FIG. 1b illustrates a state where the occlusion device illustrated in FIG. 1a occludes a tear in a tissue.
FIG. 2a is a partial schematic structural view of an elastically deformable member according to an embodiment of the disclosure.
FIG. 2b is a partial schematic structural view of an elastically deformable member 130 according to an embodiment of the disclosure.
FIG. 3 is a partial schematic structural view of an elastically deformable member according to an embodiment of the disclosure.
FIG. 4a is a partial schematic structural view of a distal locking portion or a proximal locking portion according to an embodiment of the disclosure.
FIG. 4b, FIG. 4c, and FIG. 4d respectively illustrate cross-sectional views of an elastically deformable member according to three embodiments, taken along line A-A in FIG. 4a.
FIG. 5 is a three-dimensional structural view of an occlusion device according to an embodiment of the disclosure.
FIG. 6a is a schematic structural view of an occlusion device according to an embodiment of the disclosure.
FIG. 6b illustrates a deflected posture of an elastically deformable member relative to a locking channel in a circumferential direction according to an embodiment of the disclosure.
FIG. 6c illustrates an aligned posture of an elastically deformable member relative to a locking channel in a circumferential direction according to an embodiment of the disclosure.
FIG. 6d illustrates a deflected posture of an elastically deformable member relative to a locking channel in a circumferential direction according to another embodiment of the disclosure.
FIG. 7a and FIG. 7b respectively illustrate pre-locking states of an occlusion device according to an embodiment of the disclosure.
FIG. 7c illustrates an ongoing locking state of an occlusion device according to an embodiment of the disclosure.
FIG. 7d illustrates a post-locking state of an occlusion device according to an embodiment of the disclosure.
FIG. 8 and FIG. 9 respectively illustrate cross-sectional views of a locking base of an occlusion device according to two embodiments of the disclosure, taken along a vertical plane.
FIG. 10a illustrates a cross-sectional view of a locking base of an occlusion device according to an embodiment of the disclosure, taken along a vertical plane.
FIG. 10b illustrates a cross-sectional view of an occlusion device according to an embodiment of the disclosure, taken along a vertical plane.
FIG. 11a, FIG. 11b, FIG. 11c, FIG. 11d, and FIG. 11e illustrate schematic structural views of an elastically deformable member according to five embodiments of the disclosure.
FIG. 12 is a schematic structural view of an occlusion device according to an embodiment of the disclosure.
FIG. 13 and FIG. 14 are schematic structural views of an occlusion device according to two embodiments of the disclosure.
FIG. 15 and FIG. 16 respectively illustrate schematic views of two operations in treating aortic dissection using an occlusion system.

Reference signs: 10-tissue; 11-tear; 12-false lumen; 13-entry tear; 14-true lumen; 100-occlusion device; 110-occlusion assembly; 111-distal occlusion portion; 112-proximal occlusion portion; 113-waist potion; 120-locking assembly; 121-distal locking portion; 122-proximal locking portion; 122A-central axis; 1221-locking channel; 1222-locking cavity; 1223-locking base; 1224-limiting hole; 130-elastically deformable member; 130a-distal connecting portion; 130b-expansion portion; 130c-proximal connecting portion; 131-support portion; 132-mesh portion; 140-traction member; 150-protrusion; 160-guide structure; 170-groove; 210-first operating member; 220-second operating member; 300-sheath.

### DETAILED DESCRIPTION

For the convenience of understanding the disclosure, a more comprehensive description of the disclosure will be provided below with reference to the accompanying drawings. The preferred embodiments of the disclosure are illustrated in the accompanying drawings. However, the disclosure can be implemented in many different forms and is not limited to the embodiments described herein. On the contrary, the purpose of providing these embodiments is to make the understanding of the disclosed content more thorough and comprehensive.

It may be noted that, when a component is referred to as "fixed to" another component, it may be directly on another component or it is also possible that there is a third component between them. When a component is considered to "connect to" another component, it may be directly connected to the another component or it is possible that there is a third component between them. On the contrary, when a component is referred to as "directly on" another component, there is no intermediate component. The terms "vertical", "horizontal", "left", "right" and similar expressions used herein are for illustrative purposes only.

The term "distal" as used herein refers to an end of a component that is farther away from the operator when that component is being operated, and "proximal" refers to an end of a component that is closer to the operator when that component is being operated. The "distal" and "proximal" in the disclosure refer to the relative positional relationship between components on the object being operated and the operator during operation. When the object is no longer being operated, "distal" and "proximal" may be understood as distinguishing different operational components and not for limiting specific orientations. That is, "distal" and "proximal" are for illustrative purposes only and do not limit the scope of protection of the present disclosure.

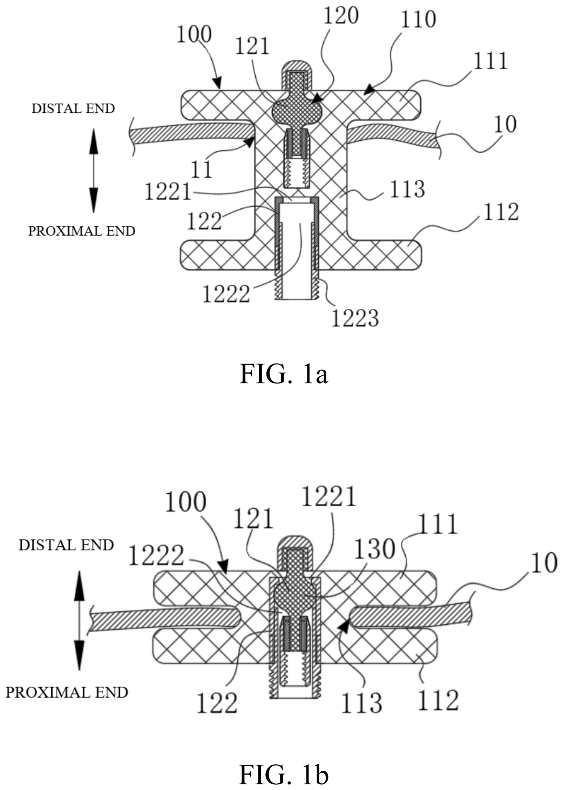

As illustrated in FIG. 1a, which is a schematic structural view of an occlusion device 100 according to an embodiment of the disclosure. FIG. 1a illustrates the occlusion device 100 during implantation into a human tissue 10. In the figure, the human tissue 10 is also illustrated, for example, the human tissue 10 is a blood vessel. The blood vessel has a tear 11. The occlusion device 100 in this embodiment is used to occlude the tear 11. To facilitate explanation of the occlusion device 100, "proximal end" and "distal end" are indicated in FIG. 1a, and "proximal end" and "distal end" may be understood as relative concepts.

The occlusion device 100 includes an occlusion assembly 110 and a locking assembly 120. The locking assembly 120 is connected to the occlusion assembly 110. The occlusion assembly 110 is extendable and contractible in an axial direction of the occlusion device 100. The axial direction of the occlusion device 100 is the vertical direction in FIG. 1a. When the occlusion assembly 110 shortens, the occlusion assembly 110 may be locked by the locking assembly 120, so that the occlusion device 100 is maintained in this shortened state, which may be referred to FIG. 1b.

As illustrated in FIG. 1a, the occlusion assembly 110 includes a distal occlusion portion 111, a waist portion 113, and a proximal occlusion portion 112. The waist portion 113 is connected between the distal occlusion portion 111 and the proximal occlusion portion 112. The distal end of the occlusion device 100 is the upper end in FIG. 1a, while the proximal end of the occlusion device 100 is the lower end in FIG. 1a. To enable the occlusion assembly 110 to occlude the tear 11 in the tissue 10, the radial dimensions of the distal occlusion portion 111 and the proximal occlusion portion 112 are greater than the radial dimension of the waist portion 113. After the occlusion assembly 110 passes through the tear 11 in the tissue 10, the waist portion 113 is located on the tear 11, while the distal occlusion portion 111 and the proximal occlusion portion 112 are located on two sides of the tissue 10. The distal occlusion portion 111 and the proximal occlusion portion 112 can shorten in the axial direction to adhere to two sides of the tissue 10, achieving occlusion of the tear 11 in the tissue 10 (see FIG. 1b).

As illustrated in FIG. 1b, which illustrates a state where the occlusion device 100 illustrated in FIG. 1a occludes the tear 11 in the tissue 10. The distal occlusion portion 111 and the proximal occlusion portion 112 respectively adhere to two sides of the tissue 10.

The occlusion assembly 110 is deformable, and is deformable at least in a radial direction and the axial direction. The radial direction in FIG. 1a refers to the horizontal direction, and the axial direction in FIG. 1a refers to the vertical direction. Radial deformation enables the distal occlusion portion 111 to contract to a size less than the size of the tear 11, allowing the distal occlusion portion 111 to pass through the tear 11 in the tissue 10. Axial deformation enables the distal occlusion portion 111 and the proximal occlusion portion 112 to adhere to the surface of the tissue 10, thereby occluding the tear 11 more securely.

As illustrated in FIG. 1a, the locking assembly 120 includes a distal locking portion 121 and a proximal locking portion 122. The distal locking portion 121 is connected to the distal occlusion portion 111, and the proximal locking portion 122 is connected to the proximal occlusion portion 112. For example, the occlusion assembly 110 is of a cage-like structure with an internal cavity. At least a portion of the distal locking portion 121 is located at the internal cavity of the cage-like structure, and at least a portion of the proximal locking portion 122 is located at the internal cavity of the cage-like structure.

In the state illustrated in FIG. 1a, the occlusion assembly 110 is in an expanded state or an elongated state in the axial direction, and the distal locking portion 121 and the proximal locking portion 122 are in a pre-locking state, or an unlocked state, or a non-locked state. In this case, the distal occlusion portion 111 and the proximal occlusion portion 112 can be operated to move closer or farther apart from each other. As illustrated in FIG. 1b, when the distal occlusion portion 111 and the proximal occlusion portion 112 move closer to each other to place the occlusion assembly 110 in a contracted state in the axial direction, mutual locking between the distal occlusion portion 111 and the proximal occlusion portion 112 can be achieved through the distal locking portion 121 and the proximal locking portion 122, so that the occlusion assembly 110 is maintained in the contracted state in the axial direction illustrated in FIG. 1b.

At least one of the distal locking portion 121 and the proximal locking portion 122 includes an elastically deformable member 130, and at least a portion of the elastically deformable member 130 is elastically deformable. That is, the elastically deformable member 130 as a whole may elastically deform, or a portion of the elastically deformable member 130 may elastically deform while another portion of the elastically deformable member 130 is rigid. Locking and unlocking between the distal occlusion portion 111 and the proximal occlusion portion 112 are achieved through elastic deformation of the elastically deformable member 130. The elastically deformable member 130 refers to a structure that inherently possesses elasticity. For example, when subjected to external compressive force, the elastically deformable member 130 may reduce in size through elastic deformation; when the external compressive force is removed, the elastically deformable member 130 may expand in size through elastic deformation. For another example, when subjected to external tensile force, the elastically deformable member 130 may expand in size through to elastic deformation; when the external tensile force is removed, the elastically deformable member 130 may reduce in size through to elastic deformation.

In some embodiments, the elastically deformable member 130 may be of a solid structure or a hollow-shell structure. A hollow-shell structure refers to a structure formed by an outer wall enclosing a cavity, where the outer wall may be continuous, or may be perforated to form a porous structure. The porous structure may be a mesh structure for example.

In some embodiments, the elastically deformable member 130 may be of a sheet-like structure or a solid structure. The solid structure may be distinguished from the sheet-like structure. For example, the solid structure may be a regular structure such as a cylinder, a sphere, a cone, or the solid structure may be other irregular structures. As illustrated in FIG. 1a and FIG. 1b, the distal locking portion 121 including the elastically deformable member 130 with at least an elastically deformable portion is taken as an example to explain the above situation. In this case, the proximal locking portion 122 may be a rigid component, and the proximal locking portion 122 may be of a tubular structure having a locking cavity 1222. A locking channel 1221 communicating between the locking cavity 1222 and the exterior of the tubular structure is defined at a distal end of the tubular structure. Moreover, the distal end of the proximal locking portion 122 generally curves inward in the radial direction, thereby making the radial dimension of the locking channel 1221 less than the radial dimension of the locking cavity 1222. When the distal locking portion 121 is not subjected to radial compressive force or axial tensile force, a region having the maximum radial dimension in the radial direction is called a first region. The size of the first region is greater than the radial dimension of the locking channel 1221 of the proximal locking portion 122, and in this case, the first region cannot pass through the locking channel 1221. When the distal locking portion 121 is contracted in the radial direction, the radial dimension of the first region is reduced to be less than the radial dimension of the locking channel 1221, thereby enabling the first region to pass through the locking channel 1221 and enter the locking cavity 1222 of the proximal locking portion 122. Thereafter, when the radial compressive force on the distal locking portion 121 is removed or the axial tensile force on the distal locking portion 121 is removed, the distal locking portion 121 may expand in size autonomously through elastic deformation. When the first region expands to a situation where the size of the first region is greater than the radial dimension of the locking channel 1221, as illustrated in FIG. 1b, locking between the distal locking portion 121 and the proximal locking portion 122 is achieved.

The radial dimension of the distal locking portion 121 may be reduced by means of compressive force applied in the radial direction or tensile force applied in the axial direction, such that the length of the distal locking portion 121 in the axial direction is increased while the radial dimension of the distal locking portion 121 is reduced.

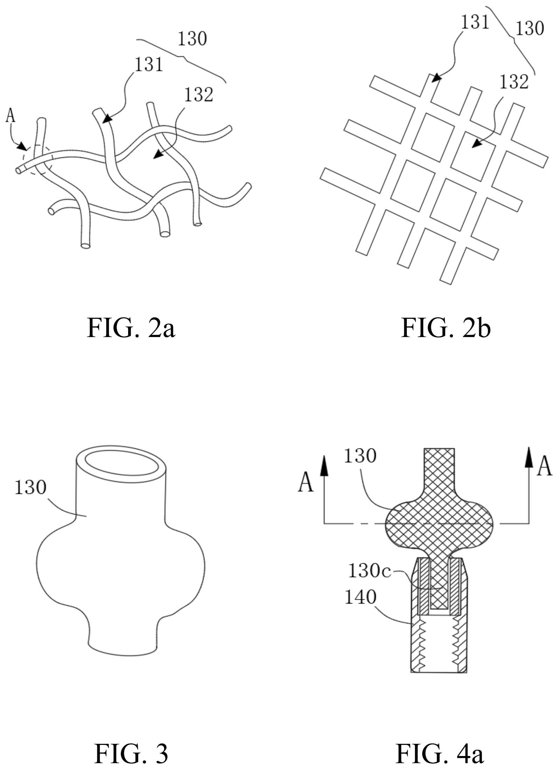

FIG. 2a is a partial schematic structural view of the elastically deformable member 130 according to an embodiment of the disclosure. The elastically deformable member 130 includes a mesh structure. That is, the elastically deformable member 130 as a whole may be of a mesh structure, or a portion of the elastically deformable member 130 is of a mesh structure and another portion of the elastically deformable member 130 is of a solid structure.

In some embodiments, the mesh structure may be understood as a structure having mesh pores. This structure may be manufactured by braiding, or may be manufactured by removing portions of material from a sheet-like structure or a plate-like structure to form mesh pores. The mesh structure manufactured by braiding may include multiple filaments arranged at intervals in a transverse direction and multiple filaments arranged at intervals in a longitudinal direction. For example, one transverse filament passes through two adjacent longitudinal filaments, and intersection points between the transverse filament and the two longitudinal filaments are located on two opposite sides of the peripheral surface of the transverse filament respectively.

In some embodiments, the mesh structure is elastically deformable. The mesh structure being elastically deformable means that the mesh structure possesses elasticity. For example, when subjected to pressure or tension, the mesh pores deform; after the applied pressure or tension is removed, the mesh structure has the ability to recover to the original shape. During elastic deformation, relative displacement exists between the crisscrossing filaments in the mesh structure manufactured by braiding, enabling the mesh structure to be compressed to a smaller volume. In an embodiment, as illustrated in FIG. 2a, the mesh structure includes a support portion 131 and a mesh portion 132. The support portion 131 primarily serves as a support, i.e., maintaining the physical shape of the elastically deformable member 130. Under external compressive or tensile force, the support portion 131 can deform elastically. Under equivalent external force, the mesh portion 132 is more prone to deformation than the support portion 131.

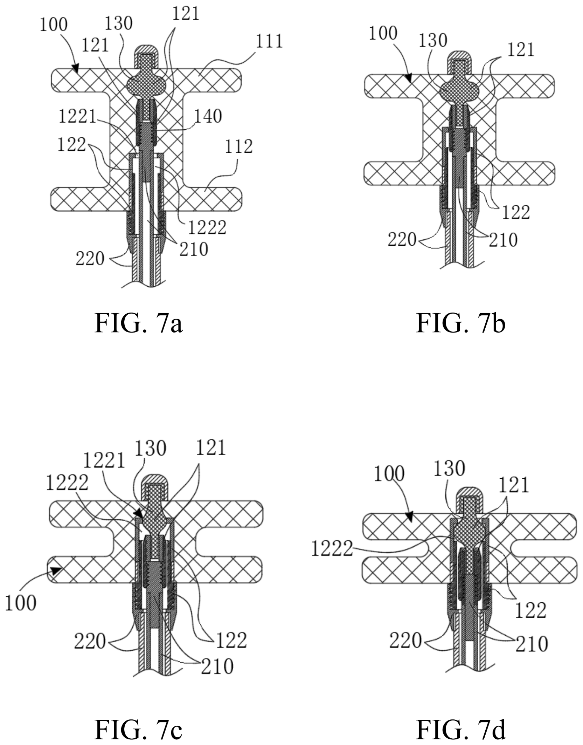

The locking process of the distal locking portion 121 and the proximal locking portion 122 is divided chronologically into three stages: pre-locking state, locking-in-progress state, and post-locking state. FIG. 7a, FIG. 7b, FIG. 7c, and FIG 7d are used to illustrate these three states. FIG. 7a and FIG. 7b illustrate the pre-locking state, FIG. 7c illustrates the locking-in-progress state, and FIG. 7d illustrates the post-locking state. The difference between FIG. 7a, FIG. 7b, FIG. 7c, FIG. 7d and FIG. 1a and FIG. 1b is that FIG. 7a, FIG. 7b, FIG. 7c, and FIG. 7d illustrate the occlusion device 100 connected to a first operating member 210 and a second operating member 220 configured to control contraction and expansion of the occlusion device 100.

As illustrated in FIG. 7a and FIG. 7b, two different phases of the pre-locking state are respectively illustrated. The axial length of the occlusion device 100 in FIG. 7b is less than the axial length of the occlusion device 100 in FIG. 7a, that is, the occlusion device 100 illustrated in FIG. 7b is in a state after partial axial compression of the occlusion device 100 illustrated in FIG. 7a. As illustrated in FIG. 7a, the distal locking portion 121 and the proximal locking portion 122 are separated from each other. As illustrated in FIG. 7b, a portion of the distal locking portion 121 is located at the proximal locking portion 122, but a portion of the distal locking portion 121 having the maximum radial dimension (i.e., the first region mentioned above) remains outside the proximal locking portion 122.

As illustrated in FIG. 7c, the locking-in-progress state may be understood as a state where the region of the distal locking portion 121 having the maximum radial dimension (i.e., the first region mentioned above) has just entered the locking channel 1221 of the proximal locking portion 122, but has not yet entered the locking cavity 1222 of the proximal locking portion 122.

As illustrated in FIG. 7d, the post-locking state may be understood as a state where the region of the distal locking portion 121 having the maximum radial dimension (i.e., the first region mentioned above) has entered the locking cavity 1222 of the proximal locking portion 122.

The elastic deformation of the elastically deformable member 130 affects the locking and unlocking between the distal locking portion 121 and the proximal locking portion 122. Specifically, in the pre-locking state illustrated in FIG. 7a and FIG. 7b, the elastically deformable member 130 is in a fully expanded state in the radial direction. In the post-locking state illustrated in FIG. 7d, the elastically deformable member 130 may be in a fully expanded state in the radial direction or may be compressed to some extent by the side walls of the locking cavity 1222 of the proximal locking portion 122. In the locking-in-progress state illustrated in FIG. 7c, the elastically deformable member 130 is further compressed in the radial direction compared to the pre-locking state illustrated in FIG. 7a and FIG. 7b, such that the elastically deformable member 130 can pass through the locking channel 1221 of the proximal locking portion 122.

As illustrated in FIG. 1b, when the occlusion device 100 occludes the tear 11 in the tissue 10, the elastically deformable member 130 in the distal locking portion 121 is located at the locking cavity 1222 of the proximal locking portion 122. The elastically deformable member 130 is in a radially expanded state and is jammed by a flange at a junction of the locking cavity 1222 and the locking channel 1221, thereby maintaining the post-locking state.

As introduced in the above embodiments, the elastically deformable member 130 has a mesh structure. The elastically deformable member 130 being designed to have a mesh structure can coagulate blood faster at the mesh structure to form blood clots, thereby enhancing the strength of the elastically deformable member 130 and facilitating the occlusion device 100 to maintain the locked state illustrated in FIG. 1b more stably.

That is, by designing the elastically deformable member 130 as a mesh structure, on one hand, the elastically deformable member 130 can be compressed to a smaller size in the radial direction in the locking process illustrated from FIG. 1a to FIG. 1b, such that the locking channel 1221 of the proximal locking portion 122 is allowed to be designed to be smaller. Therefore, miniaturization of the occlusion device 100, the first operating member 210, and the second operating member 220 is facilitated, enabling passage through smaller-diameter blood vessels. Additionally, the size of the portion of the elastically deformable member 130 having the maximum radial dimension when passing through the locking channel 1221 changes more substantially relative to the size of the portion of the elastically deformable member 130 having the maximum radial dimension when expanding in the locking cavity 1222 of the proximal locking portion 122, making the locked state of the occlusion device 100 illustrated in FIG. 1b more secure. On the other hand, in the locked state illustrated in FIG. 1b, blood may be coagulated faster to form blood clots in the elastically deformable member 130, thereby enhancing the strength of the elastically deformable member 130 in the locked state. Therefore, it is difficult for the elastically deformable member 130 to pass through the locking channel 1221 of the proximal locking portion 122 reversely, thereby improving the locking strength of the proximal locking portion 122 and distal locking portion 121.

In an embodiment, as illustrated in FIG. 2a, the support portion 131 includes multiple support rods, the multiple support rods are braided to form the mesh structure, and at least two support rods at each of braiding intersection points are slidable relative to each other. In some embodiments, the support rods may be made of elastic material, which may be metal material or non-metal material. For example, the support rods may be elastic metal wires. Circular frame A in FIG. 2a illustrates a braiding intersection point of two support rods. At this braiding intersection point, the two support rods are in contact but not fixedly connected to each other. Therefore, when the elastically deformable member 130 deforms, the two support rods at this braiding intersection point can adaptively slide relative to each other in the axial direction, circumferential direction, and other directions. The two support rods may even slide to a nearly parallel state. The design allows the elastically deformable member 130 to easily contract to a smaller size. On one hand, the locking channel 1221 of the proximal locking portion 122 may be designed to be smaller, facilitating miniaturization. On the other hand, the size of the elastically deformable member 130 when passing through the locking channel 1221 changes more substantially relative to the size of the elastically deformable member 130 when expanding in the locking cavity 1222 of the proximal locking portion 122, thereby improving the locking strength of the proximal locking portion 122 and distal locking portion 121.

FIG. 2b is a partial schematic structural view of the elastically deformable member 130 according to an embodiment of the disclosure. The support portion 131 includes a mesh structure formed by fixedly connecting multiple support rods. For example, a metal with good elasticity, such as a nitinol tube, can be processed by cutting to form multiple mesh pores. The shape of the mesh pores is not limited to the illustrated quadrilateral, but may also be circular, elliptical, triangular, or other polygons. The support portion 131 formed by cutting has better radial support force, thereby having better expansibility. Therefore, in the locked state of the occlusion device 100 illustrated in FIG. 1b, the elastically deformable member 130 is not easily compressed in the radial direction to pass through the locking channel 1221 of the proximal occlusion portion 112 reversely, avoiding locking failure of the distal occlusion portion 111 and the proximal occlusion portion 112. Consequently, the elastically deformable member 130 may be more stably positioned at the locking cavity 1222 of the proximal locking portion 122, thereby improving the locking strength of the proximal locking portion 122 and distal locking portion 121.

In some embodiments, the elastically deformable member 130 may be of a sheet-like structure extendable in a plane, or a tubular-like structure rotatable in three-dimensional space. However, the tubular structure may have both ends open or both ends closed.

For example, FIG. 3 is a partial schematic structural view of the elastically deformable member 130 according to an embodiment of the disclosure. The elastically deformable member 130 is of a tubular-like structure having an internal cavity. Since the elastically deformable member 130 has an internal cavity, the internal cavity may be flattened when the elastically deformable member 130 is subjected to radial compressive force, such that the elastically deformable member 130 may be easily contracted to a smaller size, thereby improving the deformation ratio or deformation extent of the elastically deformable member 130 in the locking-in-progress state and the post-locking state. However, although the contractibility of the elastically deformable member 130 may be improved by defining an internal cavity, it does not mean that other components cannot be disposed inside the internal cavity, as long as there is a space within the elastically deformable member 130 that can be compressed.

FIG. 4a is a partial schematic structural view of the distal locking portion 121 or the proximal locking portion 122 according to an embodiment of the disclosure. At least one of the distal locking portion 121 or the proximal locking portion 122 includes a traction member 140, and the traction member 140 is connected to at least one of the internal cavity of the elastically deformable member 130 or an exterior of the elastically deformable member 130. In this embodiment, the traction member 140 is connected to the exterior of the elastically deformable member 130. The traction member 140 is configured to detachably connect to a component for operating the movement of the elastically deformable member 130. That is, an operator can apply axial tensile force to the elastically deformable member 130 by pulling the component, thereby axially elongating and radially contracting the elastically deformable member 130. For example, in FIG. 7a, the traction member 140 is detachably connected to the first operating member 210.

Since the traction member 140 is disposed at the exterior of the elastically deformable member 130 having an internal cavity, the elastically deformable member 130 is allowed to more fully contract in the radial direction. Therefore, the elastically deformable member 130 is allowed to easily contract to a smaller size, thereby improving the deformation ratio or deformation extent of the elastically deformable member 130 in the locking-in-progress state and the post-locking state, thus enhancing the locking strength of the proximal locking portion 122 and the distal locking portion 121.

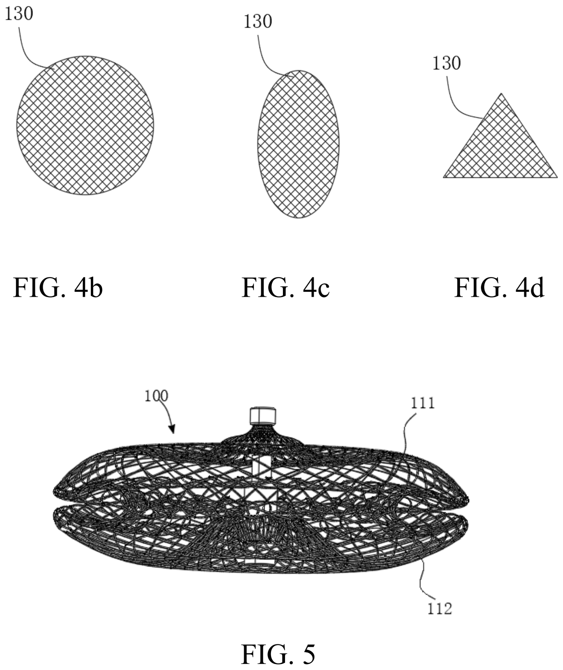

FIG. 5 is a three-dimensional structural view of the occlusion device 100 according to an embodiment of the disclosure. The occlusion device 100 includes the distal occlusion portion 111 and the proximal occlusion portion 112. As can be seen in FIG. 5, the occlusion device 100 overall is of a mesh cage-like structure formed by the support rods. A portion between the distal occlusion portion 111 and the proximal occlusion portion 112 in the axial direction is the waist portion 113. A lateral peripheral region corresponding to the waist portion 113 may be an inwardly recessed region on the lateral periphery of the occlusion device 100 between the distal occlusion portion 111 and the proximal occlusion portion 112. When the occlusion device 100 occludes the tear 11 in the tissue 10, the waist portion 113 can pass through the tear 11 in the tissue 10, and the distal occlusion portion 111 and the proximal occlusion portion 112 are located at both sides of the tissue 10 at the edge of the tear 11. The distal occlusion portion 111 and the proximal occlusion portion 112 can clamp both sides of a tissue wall around the tear 11. Furthermore, a better occlusion is achieved by arranging a membrane structure on the support rods to prevent blood from flowing through the tear 11, where the membrane structure may prevent blood from flowing to a certain extent. The radial dimension of the waist portion 113 is less than the redial dimensions of the distal occlusion portion 111 and the proximal occlusion portion 112, such that the firmness of the occlusion device 100 mounted on the tear 11 in the tissue 10 is enhanced, and the membrane structure may be prevented from tearing to a certain extent. The proximal locking portion 122 and the distal locking portion 121 are mechanically locked, such that the axial direction of the occlusion device 100 is aligned with the axial direction of the tear 11 in the tissue 10, making it less likely for the distal occlusion portion 111 and the proximal occlusion portion 112 to warp relative to the surface of the tissue 10, thereby improving the occlusion effect.

In embodiments of the disclosure, one of the distal locking portion 121 and the proximal locking portion 122 includes the elastically deformable member 130, and at least a portion of the elastically deformable member 130 is elastically deformable. The other of the distal locking portion 121 and the proximal locking portion 122 includes a locking base 1223. Locking and unlocking between the elastically deformable member 130 and the locking base 1223 are achieved through elastic deformation of the elastically deformable member 130.

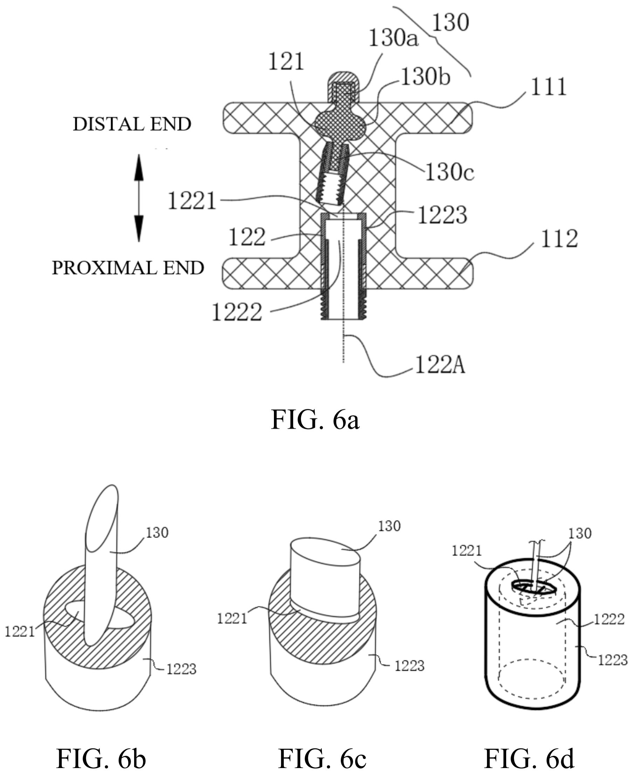

FIG. 6a is a schematic structural view of the occlusion device 100 according to an embodiment of the disclosure. An example where the distal locking portion 121 includes the elastically deformable member 130, at least a portion of the elastically deformable member 130 is elastically deformable, and the proximal locking portion 122 includes the locking base 1223 is taken to explain the occlusion device 100.

As illustrated in FIG. 6a, the locking cavity 1222 and the locking channel 1221 communicating with the locking cavity 1222 are defined in the locking base 1223. The elastically deformable member 130 can enter the locking cavity 1222 via the locking channel 1221, thereby enabling the distal locking portion 121 to be locked into the proximal locking portion 122. The maximum inner diameter of the locking cavity 1222 is greater than the maximum inner diameter of the locking channel 1221, which requires the elastically deformable member 130 to be radially compressed to pass through the locking channel 1221 with a smaller inner diameter. After the elastically deformable member 130 reaches the locking cavity 1222, the elastically deformable member 130 can self-expand. When at least a portion of the elastically deformable member 130 self-expands to a size greater than the inner diameter of the locking channel 1221, this portion of the elastically deformable member 130 can be stably positioned at the locking cavity 1222. Therefore, after at least a portion of the elastically deformable member 130 radially contracts and passes through the locking channel 1221, the portion of the elastically deformable member 130 that has passed through the locking channel 1221 expands radially in the locking cavity 1222, thereby achieving locking between the distal locking portion 121 and the proximal locking portion 122.

Reference is again made to FIG. 6a, the elastically deformable member 130 is deflected relative to the locking channel 1221. The proximal end of the elastically deformable member 130 is not precisely aligned with the locking channel 1221 but is deflected relative to the locking channel 1221 by a distance in the radial direction of the occlusion device 100. The meaning of "the elastically deformable member 130 is deflected relative to the locking channel 1221" may also be understood as follows. FIG. 6a illustrates a central axis 122A of the locking channel 1221, which extends in the axial direction of the occlusion device 100. Extending the central axis 122A towards the distal end of the occlusion device 100, the central axis 122A will pass through the distal end of the elastically deformable member 130. Connecting the proximal end and the distal end of the elastically deformable member 130 with a straight line reveals that the straight line forms an angle less than 90° with the central axis 122A.

It is to be further noted that the above-mentioned "the elastically deformable member 130 is deflected relative to the locking channel 1221" refers to a natural state of the elastically deformable member 130 without the constraint of external forces. Since the elastically deformable member 130 has elasticity, the deflected posture may be changed when the elastically deformable member 130 is subjected to an external force. However, elastic stress is generated in the elastically deformable member 130 after the deflected posture is changed. The elastic stress causes the elastically deformable member 130 to restore the aforementioned deflected posture once the external force is removed.

For example, in FIG. 7a, at the proximal end of the distal locking portion 121, the first operating member 210 is detachably connected. The first operating member 210 passes through the locking channel 1221 and the locking cavity 1222 of the proximal locking portion 122. The first operating member 210 applies an external force constraint to the elastically deformable member 130 of the distal locking portion 121. The external force constraint causes the elastically deformable member 130 to no longer be deflected relative to the locking channel 1221 but instead aligned with the locking channel 1221. The elastically deformable member 130 can then enter the locking channel 1221 by axial elongation.

However, in FIG. 7a, since the first operating member 210 applies an external force constraint to the elastically deformable member 130, elastic stress is generated in the elastically deformable member 130. This elastic stress causes the elastically deformable member 130 to self-restore to the deflected state illustrated in FIG. 6a after the first operating member 210 is separated from the distal locking portion 121. In this arrangement, as illustrated in FIG. 1b, under the stress generated by the deflection of the elastically deformable member 130, when the elastically deformable member 130 is located at the locking cavity 1222 of the proximal locking portion 122, in the state illustrated in FIG. 1b, the elastically deformable member 130 has a tendency to deflect leftward under the stress. As a result, the elastically deformable member 130 tightly abuts against the left inner wall of the locking cavity 1222, making it difficult for the elastically deformable member 130 to pass upward through the locking channel 1221. Consequently, the elastically deformable member 130 is more stably retained in the locking cavity 1222, thereby improving the stability of the locked state of the proximal locking portion 122 and the distal locking portion 121.

It may be understood that, in some embodiments, the deflected angle of the elastically deformable member 130 may also be adjusted. For example, the deflected angle of the elastically deformable member 130 may be adjusted so that the elastically deformable member 130 has a tendency to deflect rightward under the stress, causing the elastically deformable member 130 to tightly abut against the right inner wall of the locking cavity 1222, thereby improving the stability of the locked state of the proximal locking portion 122 and the distal locking portion 121.

FIG. 4b, FIG. 4c, and FIG. 4d respectively illustrate cross-sectional views of the elastically deformable member 130 according to three embodiments, taken along line A-A in FIG. 4a. The cross-section illustrated in FIG. 4b is circular, the cross-section illustrated in FIG. 4c is elliptical, and the cross-section illustrated in FIG. 4d is triangular. However, in other embodiments, the cross-section of the elastically deformable member 130 may be other shapes. Similarly, the cross-section of the locking channel 1221 taken along a plane parallel to the line A-A may also be circular, elliptical, triangular, or other shapes.

The elastically deformable member 130 has a first posture and a second posture. The elastically deformable member 130 is circumferentially rotatable relative to the locking channel 1221 from the first posture to the second posture. In the first posture of the elastically deformable member 130, a projection of the elastically deformable member 130 is located within a projection of the locking channel 1221 in an axial direction of the occlusion device. In the second posture of the elastically deformable member 130, the projection of the elastically deformable member 130 intersects with the projection of the locking channel 1221 in the axial direction of the occlusion device.

For example, in an embodiment, the elastically deformable member 130 has a deflected posture (second posture) and an aligned posture (first posture) relative to the locking channel 1221 in the circumferential direction, which applies to a situation where both the aforementioned cross-section of the elastically deformable member 130 and the cross-section of the locking channel 1221 are non-circular. Both cross-sections of the elastically deformable member 130 and the locking channel 1221 being elliptical is taken as an example. The "aligned posture" means that the major axis of the cross-section of the elastically deformable member 130 is parallel to the major axis of the cross-section of the locking channel 1221, and the minor axis of the cross-section of the elastically deformable member 130 is parallel to the minor axis of the cross-section of the locking channel 1221. Additionally, in the axial direction of the occlusion device, the projection of the elastically deformable member 130 is located within the projection of the locking channel 1221. The "deflected posture" means that the major axis of the cross-section of the elastically deformable member 130 is not parallel to the major axis of the cross-section of the locking channel 1221, or the minor axis of the cross-section of the elastically deformable member 130 is not parallel to the minor axis of the cross-section of the locking channel 1221. Additionally, in the axial direction of the occlusion device, the projection of the elastically deformable member 130 intersects with the projection of the locking channel 1221.

In the aligned posture, the portion of the elastically deformable member 130 having the maximum radial dimension passing through the locking channel 1221 is facilitated. In the deflected posture, the portion of the elastically deformable member 130 having the maximum radial dimension is prevented from passing through the locking channel 1221 to a certain extent.

FIG. 6b illustrates a deflected posture of the elastically deformable member 130 relative to the locking channel 1221 in the circumferential direction according to an embodiment of the disclosure, with the elastically deformable member 130 located outside the locking base 1223. FIG. 6c illustrates an aligned posture of the elastically deformable member 130 relative to the locking channel 1221 in the circumferential direction according to an embodiment of the disclosure. FIG. 6d illustrates a deflected posture of the elastically deformable member 130 relative to the locking channel 1221 in the circumferential direction according to another embodiment of the disclosure, with the elastically deformable member 130 located inside the locking base 1223.

Without external force applied, the elastically deformable member 130 is in the deflected posture illustrated in FIG. 6b. When an external force causes the elastically deformable member 130 to deflect in the circumferential direction, the elastically deformable member 130 is made to be in the aligned posture illustrated in FIG. 6c. After removing the external force, the elastically deformable member 130 can rotate circumferentially under its own elasticity and restore the deflected posture illustrated in FIG. 6d. As illustrated in FIG. 6c, the aligned posture allows the elastically deformable member 130 to pass through the locking channel 1221. As illustrated in FIG. 6d, in the deflected posture, the portion of the elastically deformable member 130 having the maximum radial dimension is prevented from passing through the locking channel 1221 to a certain extent, making it difficult for the elastically deformable member 130 to pass upward through the locking channel 1221. Consequently, the elastically deformable member 130 is more stably retained in the locking cavity 1222, thereby improving the stability of the locked state of the proximal locking portion 122 and the distal locking portion 121.

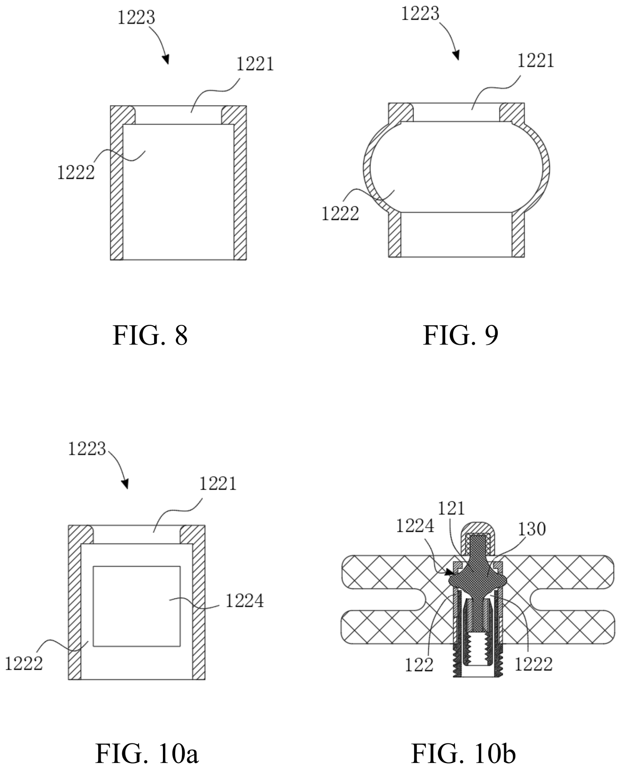

FIG. 8 and FIG. 9 respectively illustrate cross-sectional views of the locking base 1223 of the occlusion device 100 according to two embodiments of the disclosure, taken along a vertical plane. As illustrated in FIG. 8, the locking channel 1221 and the locking cavity 1222 are defined in the locking base 1223. The locking cavity 1222 communicates with the locking channel 1221. In the axial direction, i.e., in the vertical direction of FIG. 8, the inner diameter of the locking cavity 1222 remains unchanged. It may be understood that, the locking cavity 1222 includes a cylindrical cavity. In this case, the locking channel 1221 may also be a cylindrical cavity. In the embodiment illustrated in FIG. 9, in the axial direction, the locking cavity 1222 bulges radially outward. That is, in the horizontal direction of FIG. 9, the central portion of the locking cavity 1222 protrudes outward. The shape of the locking cavity 1222, in cooperation with the adaptable deformation of the elastically deformable member 130 in the axial direction, ensures that the elastically deformable member 130 is more stably retained in the locking cavity 1222, making it difficult for the elastically deformable member 130 to pass upward through the locking channel 1221. Consequently, the elastically deformable member 130 is more stably retained in the locking cavity 1222, thereby improving the stability of the locked state of the proximal locking portion 122 and the distal locking portion 121.

FIG. 10a illustrates a cross-sectional view of the locking base 1223 of the occlusion device 100 according to an embodiment of the disclosure, taken along a vertical plane. FIG. 10b illustrates a cross-sectional view of the occlusion device 100 according to an embodiment of the disclosure, taken along a vertical plane. As illustrated in FIG. 10a, a limiting hole 1224 is defined in a side wall of the locking base 1223. As illustrated in FIG. 10b, in the locked state, at least a portion of the elastically deformable member 130 passes through the limiting hole 1224 and is located outside the locking cavity 1222. Consequently, the elastically deformable member 130 is more stably retained in the locking cavity 1222, thereby improving the stability of the locked state of the proximal locking portion 122 and the distal locking portion 121.

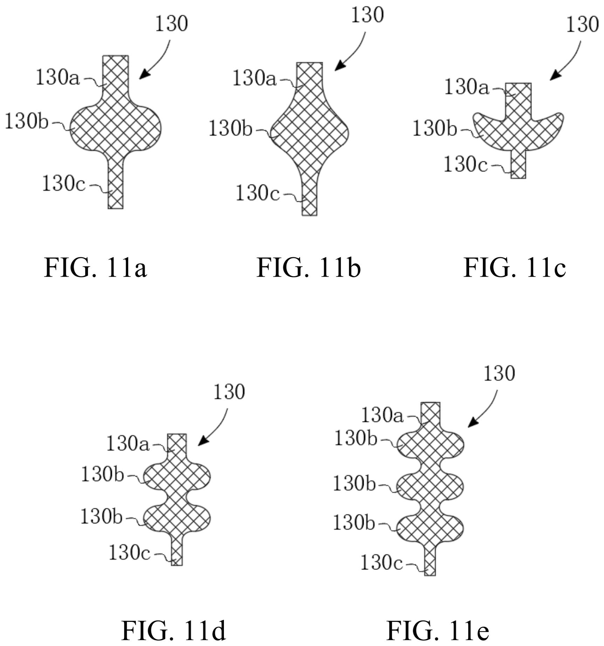

FIG. 11a, FIG. 11b, FIG. 11c, FIG. 11d, and FIG. 11e respectively illustrate schematic structural views of the elastically deformable member 130 according to five embodiments of the disclosure. The elastically deformable member 130 includes a distal connecting portion 130a, an expansion portion 130b, and a proximal connecting portion 130c. As illustrated in FIG. 6a, the distal connecting portion 130a is connected to the distal occlusion portion 111, the expansion portion 130b is connected to the proximal end of the distal connecting portion 130a, and the proximal connecting portion 130c is connected to the proximal end of the expansion portion 130b. In a natural state, where the "natural state" refers to the naturally expanded state of the expansion portion 130b without the constraint of external forces, the maximum radial dimension of the expansion portion 130b is greater than the maximum radial dimension of the distal connection portion 130a. In one embodiment, in the natural state, the expansion portion 130b is spherical. As illustrated in FIG. 11a, in the natural state, the expansion portion 130b is oblate spherical. When the distal connection portion 130a and the proximal connection portion 130c are subjected to axial tensile force, the expansion portion 130b contracts radially inward. When the tensile force is removed, the expansion portion 130b expands under its own elasticity. In the case where other factors such as radial dimension and material are identical, when compressing a oblate spherical expansion portion 130b and a spherical expansion portion 130b to the same diameter in the radial direction, the force required to compress the oblate spherical expansion portion 130b is greater than the force required to compress the spherical expansion portion 130b. Therefore, the spherical expansion portion 130b provides better locking firmness between the distal locking portion 121 and the proximal locking portion 122.

In an embodiment, as illustrated in FIG. 11c, in the natural state, the expansion portion 130b being of a concave shape curves from a central portion to an edge towards the distal occlusion portion 111. When the distal locking portion 121 and the proximal locking portion 122 are in the locked state, the expansion portion 130b is in an upward-bending shape illustrated in FIG. 11c. In this case, in order to unlock the distal locking portion 121 from the proximal locking portion 122, the expansion portion 130b is required to change to a downward-bending shape. The changing process may be achieved by a greater external force. Therefore, the design enhances the locking firmness between the distal locking portion 121 and the proximal locking portion 122.

To further improve the locking firmness between the distal locking portion 121 and the proximal locking portion 122, as illustrated in FIG. 11d and FIG. 11e, the expansion portion 130b is implemented as multiple expansion portions 130b, and the multiple expansion portions 130b are arranged in the axial direction.

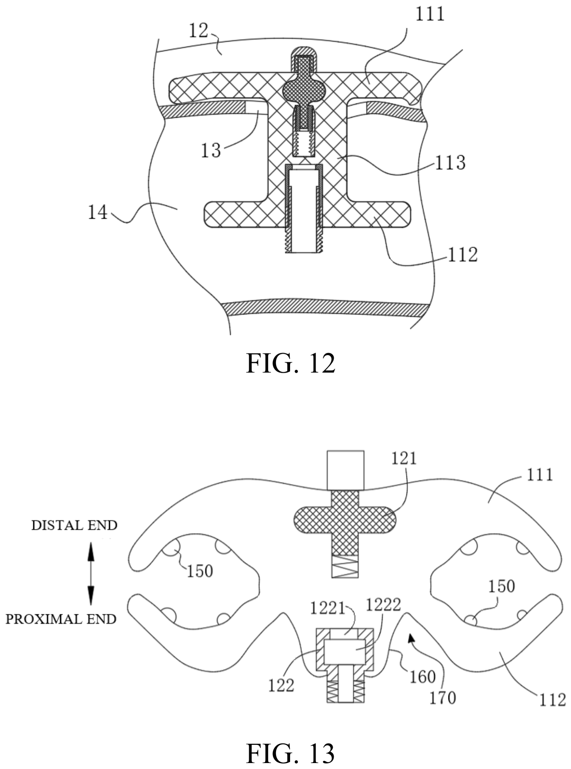

As illustrated in FIG. 12, which is a schematic structural view of the occlusion device 100 according to an embodiment of the disclosure. For example, when an intima of a blood vessel ruptures to create a false lumen 12 in a vessel wall, the distal occlusion portion 111 may be positioned in the false lumen 12, and the waist portion 113 passes through an entry tear 13 in intima of the blood vessel. The distal occlusion portion 111 is located in the false lumen 12 of the blood vessel, and the proximal occlusion portion 112 is located in a true lumen 14 of the blood vessel. Occlusion of the entry tear 13 can be achieved by the distal occlusion portion 111 and the proximal occlusion portion 112. The distal occlusion portion 111, the waist portion 113, and the proximal occlusion portion 112 of the occlusion device 100 are integrally formed. That is, the distal occlusion portion 111, the waist portion 113, and the proximal occlusion portion 112 are integrated as a whole, which improves the strength of the distal occlusion portion 111, the waist portion 113, and the proximal occlusion portion 112. The radial dimension of the waist portion 113 is less than the radial dimensions of the distal occlusion portion 111 and the proximal occlusion portion 112.

As illustrated in FIG. 12, the diameter of the distal occlusion portion 111 is greater than the diameter of the proximal occlusion portion 112. When the occlusion device 100 is released, the distal occlusion portion 111 and the proximal occlusion portion 112 are in a compressed state in the radial direction, for example, compressed in a sheath 300. The distal end of the sheath 300 extends from the true lumen 14 of the blood vessel through the entry tear 13 in the intima of the blood vessel into the false lumen 12. The distal occlusion portion 111 is then extended from the sheath 300, and the distal occlusion portion 111 self-expands in the radial direction. The occlusion device 100 is then pulled to the proximal end, so that the distal occlusion portion 111 adheres to the vessel wall. Due to the larger size of the distal occlusion portion 111, it is less likely for the distal occlusion portion 111 to pass through the entry tear 13 during pulling, preventing the distal occlusion portion 111 from being pulled out from the false lumen 12 during releasing. The sheath 300 is then pulled to the proximal end, so that the proximal occlusion portion 112 is released in the true lumen 14. Consequently, the locking between the distal locking portion 121 and the proximal locking portion 122 may be operated.

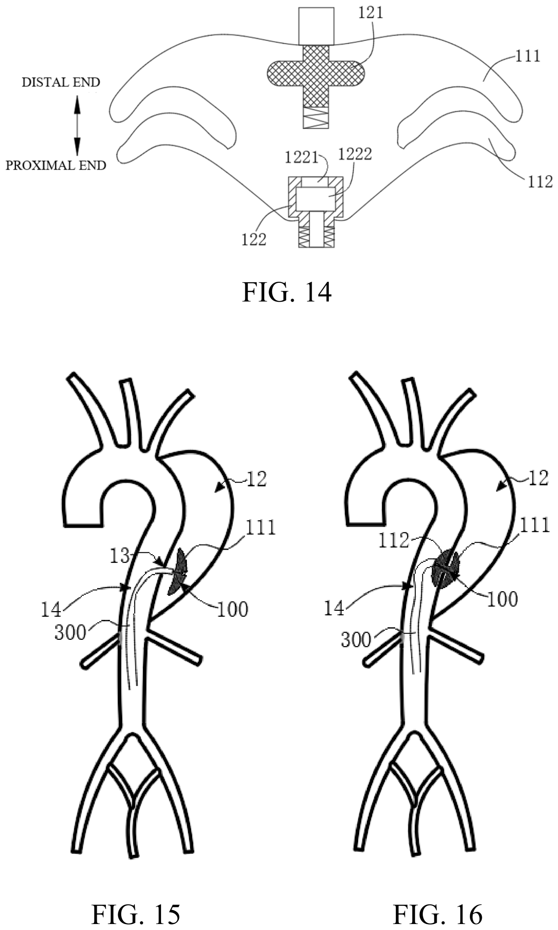

FIG. 13 and FIG. 14 are schematic structural views of the occlusion device 100 according to two embodiments of the disclosure. As illustrated in FIG. 13, the peripheral portion of the distal occlusion portion 111 bends towards the proximal end of the occlusion device 100 or the proximal occlusion portion 112, and the peripheral portion of the proximal occlusion portion 112 bends towards the distal end of the occlusion device 100 or the distal occlusion portion 111. As illustrated in FIG. 14, the peripheral portion of the distal occlusion portion 111 bends towards the proximal end of the occlusion device 100 or the proximal occlusion portion 112, and the peripheral portion of the proximal occlusion portion 112 bends towards the proximal end of the occlusion device 100. However, in other embodiments, the peripheral portion of the distal occlusion portion 111 may bend towards the distal end of the occlusion device 100, and the peripheral portion of the proximal occlusion portion 112 may bend towards the proximal end of the occlusion device 100. In other embodiments, the peripheral portion of the distal occlusion portion 111 may bend towards the distal end of the occlusion device 100, and the peripheral portion of the proximal occlusion portion 112 may bend towards the distal end of the occlusion device 100. In this arrangement, when the distal occlusion portion 111 and the proximal occlusion portion 112 adhere to the surface of the tissue 10, the distal occlusion portion 111 and the proximal occlusion portion 112 elastically deform to match the shape of the tissue 10 surface. Therefore, a greater conforming force may be exerted on the surface of the tissue 10, increasing the clamping force of the distal occlusion portion 111 and the proximal occlusion portion 112 on the tissue 10, thereby enabling the occlusion device 100 to be more firmly positioned at the target location. Moreover, the endothelialization process of the surfaces of the distal occlusion portion 111 and the proximal occlusion portion 112 may be accelerated, thereby improving the firmness of the occlusion device 100 in occluding the entry tear 13.

As illustrated in FIG. 13, a protrusion 150 is disposed at the surface of the distal occlusion portion 111 close to the proximal occlusion portion 112 and is disposed at the surface of the proximal occlusion portion 112 close to the distal occlusion portion 111. The protrusion 150 is configured to compress the surface of the tissue 10, further strengthening the clamping force of the distal occlusion portion 111 and the proximal occlusion portion 112 on the tissue 10.

One implementation of the protrusion 150 is that the protrusion 150 is disposed circumferentially in a closed loop at least around the edge of the entry tear 13 of the blood vessel. In some embodiments, multiple nested rings of protrusions 150 may be disposed.

Another implementation of the protrusion 150 is that the protrusion 150 is implemented as multiple protrusions 150, which are arranged in a dot-matrix pattern on occlusion portions.

In an embodiment of the disclosure, the occlusion device 100 adopts a retrievable design. As illustrated in FIG. 13, a conical guiding structure 160 is disposed at the proximal end of the proximal occlusion portion 112. From the proximal end to the distal end of the occlusion device 100, the outer diameter of the guiding structure 160 gradually increases. When the position of the occlusion device 100 needed to be more precisely adjusted, the occlusion device 100 can be retracted into the sheath 300 for second release. Since the guiding structure 160 has a guiding function, the occlusion device 100 can easily enter the sheath 300, making the retrievable design of the occlusion device 100 safer and more controllable. The occlusion device 100 has thin walls and good compliance, allowing position and shape adjustment before release for more precise operation and positioning.

As illustrated in FIG. 13, a groove 170 is defined in the proximal occlusion portion 112, and at least a portion of the guiding structure 160 is located in the groove 170. At least a portion of the guiding structure 160 is accommodated in the groove 170, such that the length of the guiding structure 160 exposed in the true lumen 14 of the blood vessel is reduced. In this way, formation of blood clots in the guiding structure 160 may be reduced, thereby further accelerating the endothelialization process of the proximal occlusion portion 112. Consequently, the entry tear 13 may be occluded by the occlusion device 100 more securely.

An embodiment of the disclosure further provides an occlusion system. As illustrated in FIG. 7a, the occlusion system includes the occlusion device 100 according to any of the previous embodiments, and further includes the first operating member 210 and the second operating member 220. Relative movement of the first operating member 210 and the second operating member 220 in different directions enables the locking and unlocking between the proximal locking portion 122 and the distal locking portion 121. Both the first operating member 210 and the second operating member 220 are of a tubular structure. The first operating member 210 is detachably connected to the distal locking portion 121, and the second operating member 220 is detachably connected to the proximal locking portion 122. The second operating member 220 is axially movable relative to the first operating member 210, to adjust a relative distance between the distal occlusion portion 111 and the proximal occlusion portion 112, adjust a relative distance between the distal locking portion 121 and the proximal locking portion 122, and adjust a degree of elastic deformation of the elastically deformable member 130.

After the occluder is released to the target position, the first operating member 210 and the second operating member 220 can be separated from the occlusion device 100. Portions of the first operating member 210 and the second operating member 220 located at the blood vessel are withdrawn from the blood vessel.

The first operating member 210 and the second operating member 220 may be flexible steel tubes or steel cables. The first operating member 210 and the second operating member 220 may be nested. The first operating member 210 may be connected to the distal locking portion 121 via threaded connection. The second operating member 220 may be connected to the proximal locking portion 122 via threaded connection.

As illustrated in FIG. 4a, the distal locking portion 121 includes a traction member 140 connected to the elastically deformable member 130. The traction member 140 is fixedly connected to the proximal connection portion 130c of the elastically deformable member 130. A threaded hole for connection to the first operating member 210 is defined in the traction member 140.

As illustrated in FIG. 1a, the locking base 1223 of the proximal locking portion 122 is provided with a threaded connection portion in the exterior of the occlusion assembly 110. The threaded connection portion is configured for threaded connection with the second operating member 220.

As illustrated in FIG. 1a, a portion of the locking base 1223 of the proximal locking portion 122 is located inside the occlusion assembly 110, and another portion of the locking base 1223 of the proximal locking portion 122 is located outside the occlusion assembly 110. With such design, the length of the locking base 1223 extending directly into the true lumen 14 of the blood vessel may be reduced, which helps to impede formation of blood clots and thus accelerating the endothelialization process of the proximal occlusion portion 112.

In a possible application scenario of the occlusion system, reference is made to FIG. 15 and FIG. 16. FIG. 15 and FIG. 16 respectively illustrate schematic views of two operations in treating aortic dissection using the occlusion system. The aortic dissection in the figures causes the formation of the entry tear 13 on the intima of the blood vessel, and the blood vessel is divided into the true lumen 14 and the false lumen 12 by the intima. When using the occlusion system, the occlusion device 100 is accommodated in the sheath 300. First, the distal end of the sheath 300 enters the false lumen 12 through the entry tear 13 from the true lumen 14. The sheath 300 is then retracted to the proximal end. As illustrated in FIG. 15, the distal occlusion portion 111 is released in the false lumen 12, such that the distal occlusion portion 111 adheres to one side of the intima. The sheath 300 is then further retracted to the proximal end. As illustrated in FIG. 16, the proximal occlusion portion 112 is released in the true lumen 14, such that the occlusion device 100 is mounted on the blood vessel to occlude the entry tear 13.

The various technical features in the above embodiments can be combined arbitrarily. For concise of the description, all possible combinations of the technical features in the above embodiments are not described. However, as long as there is no contradiction in the combination of these technical features, all those combinations should be considered to be within the range of this description.

The above-mentioned embodiments only express a few implementation modes of the disclosure, and their descriptions are specific and detailed, but they should not be interpreted as a limitation on the scope of patents. It should be pointed out that for one of ordinary skill in the art, several modifications and improvements can be made without departing from the concept of the disclosure. These modifications and improvements all fall within the protection scope of the disclosure. Therefore, the protection scope of the disclosure shall be subject to the appended claims.

## Claims

1. An occlusion device (100), comprising:
an occlusion assembly (110) comprising a distal occlusion portion (111), a waist portion (113), and a proximal occlusion portion (112), wherein the waist portion (113) is connected between the distal occlusion portion (111) and the proximal occlusion portion (112); and
a locking assembly (120) comprising a distal locking portion (121) and a proximal locking portion (122), wherein the distal locking portion (121) is connected to the distal occlusion portion (111), and the proximal locking portion (122) is connected to the proximal occlusion portion (112); any one of the distal locking portion (121) and the proximal locking portion (122) comprises an elastically deformable member (130), at least a portion of the elastically deformable member (130) is elastically deformable, and locking and unlocking between the distal locking portion (121) and the proximal locking portion (122) are achieved through elastic deformation of the elastically deformable member (130); and the elastically deformable member (130) comprises a mesh structure.

2. The occlusion device (100) of claim 1, wherein the elastically deformable member (130) comprises a plurality of support rods fixedly connected to one another to form the mesh structure.

3. The occlusion device (100) of claim 1, wherein the elastically deformable member (130) comprises a plurality of support rods, the plurality of support rods are braided to form the mesh structure, and at least two support rods at each of braiding intersection points are slidable relative to each other.

4. The occlusion device (100) of claim 1, wherein the elastically deformable member (130) is a structure having an internal cavity.

5. The occlusion device (100) of claim 4, wherein at least one of the distal locking portion (121) or the proximal locking portion (122) comprises a traction member (140), and the traction member (140) is connected to at least one of the internal cavity of the elastically deformable member (130) or an exterior of the elastically deformable member (130).

6. The occlusion device (100) of claim 1, wherein another one of the distal locking portion (121) and the proximal locking portion (122) comprises a locking base (1223), a locking cavity (1222) and a locking channel (1221) communicating with the locking cavity (1222) are defined in the locking base (1223), and a maximum inner diameter of the locking cavity (1222) is greater than a maximum inner diameter of the locking channel (1221); and the elastically deformable member (130) is radially contractible and is configured to pass through the locking channel (1221), and at least a portion of the elastically deformable member (130) is radially expandable in the locking cavity (1222) to lock the distal locking portion (121) and the proximal locking portion (122).

7. The occlusion device (100) of claim 6, wherein the elastically deformable member (130) is deflected relative to a central axis of the locking channel (1221).

8. The occlusion device (100) of claim 6, wherein the elastically deformable member (130) has a first posture and a second posture, and the elastically deformable member (130) is circumferentially rotatable relative to the locking channel (1221) from the first posture to the second posture;
in the first posture of the elastically deformable member (130), a projection of the elastically deformable member (130) is located within a projection of the locking channel (1221) in an axial direction of the occlusion device (100); and
in the second posture of the elastically deformable member (130), the projection of the elastically deformable member (130) intersects with the projection of the locking channel (1221) in the axial direction of the occlusion device (100).

9. The occlusion device (100) of claim 6, wherein in an axial direction, the locking cavity (1222) is at least one of a cylindrical cavity or a cavity having a radially outward protrusion (150).

10. The occlusion device (100) of claim 6, wherein a limiting hole (1224) is defined in a side wall of the locking base (1223), and in a locked state, at least a portion of the elastically deformable member (130) passes through the limiting hole (1224) and is located outside the locking cavity (1222).

11. The occlusion device (100) of claim 1, wherein the elastically deformable member (130) comprises a distal connecting portion (130a) and an expansion portion (130b), the distal connecting portion (130a) is connected to the distal occlusion portion (111), and a maximum radial dimension of the expansion portion (130b) is greater than a maximum radial dimension of the distal connection portion (130a).

12. The occlusion device (100) of claim 11, wherein the expansion portion (130b) is of at least one of a spherical shape, an oblate spherical shape, or a concave shape, and the expansion portion (130b) being of the concave shape curves from a central portion to an edge towards the distal occlusion portion (111).

13. The occlusion device (100) of claim 11, wherein the expansion portion (130b) is implemented as a plurality of expansion portions (130b), and the plurality of expansion portions (130b) are arranged in an axial direction.

14. The occlusion device (100) of claim 1, wherein the distal occlusion portion (111), the waist portion (113), and the proximal occlusion portion (112) are integrally formed, and a radial dimension of the waist portion (113) is less than radial dimensions of the distal occlusion portion (111) and the proximal occlusion portion (112).

15. The occlusion device (100) of claim 1, wherein a diameter of the distal occlusion portion (111) is greater than a diameter of the proximal occlusion portion (112).

16. The occlusion device (100) of claim 1, wherein a peripheral portion of the distal occlusion portion (111) bends towards at least one of a proximal end or a distal end of the occlusion device (100); and/or, a peripheral portion of the proximal occlusion portion (112) bends towards at least one of the proximal end or the distal end of the occlusion device (100).

17. The occlusion device (100) of claim 1, wherein a protrusion (150) is disposed at at least one of one side of the distal occlusion portion (111) close to the proximal occlusion portion (112) or one side of the proximal occlusion portion (112) close to the distal occlusion portion (111).

18. The occlusion device (100) of claim 1, wherein a conical guiding structure (160) is disposed at a proximal end of the proximal occlusion portion (112).

19. The occlusion device (100) of claim 18, wherein a groove (170) is defined in the proximal occlusion portion (112), and at least a portion of the guiding structure (160) is located in the groove (170).

20. An occlusion system, comprising:
the occlusion device (100) of any one of claims 1 to 19;
a first operating member (210) connected to the distal locking portion (121); and
a second operating member (220) connected to the proximal locking portion (122), wherein the second operating member (220) is axially movable relative to the first operating member (210), to adjust a relative distance between the distal occlusion portion (111) and the proximal occlusion portion (112), adjust a relative distance between the distal locking portion (121) and the proximal locking portion (122), and adjust a degree of elastic deformation of the elastically deformable member (130).

21. The occlusion system of claim 20, wherein the first operating member (210) is detachably connected to the distal locking portion (121), and the second operating member (220) is detachably connected to the proximal locking portion (122).
